# EUROPEAN PATENT APPLICATION

(11) **EP 3 501 375 A1**
(43) Date of publication of application: **26.06.2019**
(21) Application number: 17209903.8
(22) Date of filing: 22.12.2017
(51) Int. Cl.: A61B 3/032

(54) **AN EYE MONITORING APPARATUS**

(71) Applicant: Nokia Technologies Oy, 02610 Espoo (FI)
(72) Inventor: SALMIMAA, Marja, 33610 Tampere (FI); JÄRVENPÄÄ, Toni, 37800 Akaa (FI); ESKOLIN, Jan Peter, 33960 Tampere (FI)
(74) Representative: Anglesea, Christine Ruth

(57) **Abstract**

An apparatus and method comprising: an image source; an optical arrangement comprising at least one light guide and an exit pupil where the exit pupil is arranged to be positioned proximate to an eye of a user and the at least one light guide is arranged to convey a beam of light from the image source to the exit pupil to enable an image from the image source to be projected onto the user's eye; a detector arranged to detect the image projected onto the user's eye; and means for enabling the detected image to be analysed so as to identify changes in the projected image.

## Description

### TECHNOLOGICAL FIELD

Examples of the disclosure relate to an eye monitoring apparatus. In examples of the disclosure images may be projected onto the user's eye to enable the eye to be monitored.

### BACKGROUND

Apparatus for monitoring a user's eye can be used to obtain information about the condition of the user's eye. These could be used to detect changes in the shape of the eye or the presence of any abrasions or other irregularities within the eye.

### BRIEF SUMMARY

According to various, but not necessarily all, examples of the disclosure there is provided an, apparatus comprising: an image source; an optical arrangement comprising at least one light guide and an exit pupil where the exit pupil is arranged to be positioned proximate to an eye of a user and the at least one light guide is arranged to convey a beam of light from the image source to the exit pupil to enable an image from the image source to be projected onto the user's eye; a detector arranged to detect the image projected onto the user's eye; and means for enabling the detected image to be analysed so as to identify changes in the projected image.

The means for enabling the detected image to be analysed may comprise image processing circuitry.

The means for enabling the detected image to be analysed may comprise one or more transceivers arranged to transmit the detected image to a remote device to enable the remote device to analyse the image.

The apparatus may be provided in a wearable device.

The image source may comprise a light source.

The image source may comprise a plurality of collimated light sources.

The image source may comprise at least one display. The display may be curved.

The image provided by the image source may comprise an array of objects.

The light guide may comprise one or more diffraction gratings. The light guide and the one or more diffraction gratings may provide an exit pupil expander.

The optical arrangement may be arranged to duplicate the image provided by the image source so that a plurality of duplicated images are projected onto the user's eye.

The diffraction gratings in the light guide element may be arranged to cause duplication of the image.

The detector may comprise an image capturing device configured to capture an image of the user's eye and the image projected onto the user's eye.

The detector may comprise a light source configured to illuminate the user's eye using infrared light.

The apparatus may comprise means for using the image detected by the detector to determine the position of the user's pupil.

The optical arrangement may comprise adjustment means arranged to adjust the position of the projected image to be aligned with the user's pupil.

According to various, but not necessarily all, examples of the disclosure there is provided an, apparatus comprising: an image source; an optical arrangement comprising at least one light guide and an exit pupil where the exit pupil is arranged to be positioned proximate to an eye of a user and the at least one light guide is arranged to convey a beam of light from the image source to the exit pupil to enable an image from the image source to be projected onto the user's eye; a detector arranged to detect the image projected onto the user's eye and enable the detected image to be analysed so as to identify changes in the projected image.

The apparatus may comprise image processing circuitry for analysing the detected image.

The apparatus may comprise one or more transceivers arranged to transmit the detected image to a remote device to enable the remote device to analyse the image.

According to various, but not necessarily all, examples of the disclosure there is provided a method comprising: providing an image from an image source; conveying a beam of light through an optical arrangement to enable an image from the image source to be projected onto the user's eye wherein the optical arrangement comprises at least one light guide and an exit pupil where the exit pupil is arranged to be positioned proximate to an eye of a user and the at least one light guide is arranged to convey a beam of light from the image source to the exit pupil; detecting the image projected onto the user's eye; and enabling the detected image to be analysed so as to identify changes in the projected image.

According to various, but not necessarily all, embodiments of the invention there is provided examples as claimed in the appended claims.

### BRIEF DESCRIPTION

For a better understanding of various examples that are useful for understanding the detailed description, reference will now be made by way of example only to the accompanying drawings in which:
Fig. 1 illustrates an apparatus;
Fig. 2 illustrates an apparatus;
Figs. 3A to 3D illustrate an example apparatus and image;
Figs. 4A to 4D illustrate an example apparatus and image;
Figs. 5A to 5D illustrate an example apparatus and image;
Figs. 6A to 6D illustrate an example apparatus and image;
Figs. 7A to 7D illustrate an example apparatus and image;
Figs. 8A to 8D illustrate an example apparatus and image;
Figs. 9A to 9D illustrate example images;
Figs. 10A and 10B illustrate images projected onto a user's eye;
Fig. 11 shows light beams required for coverage of the user's cornea
Fig. 12 shows the coverage of the user's cornea obtained with a narrow light beam;
Figs. 13A to 13C show apparatus arranged to obtain extended coverage of the cornea of the user's eye;
Figs. 14A to 14C show diffraction gratings arranged to obtain extended coverage of the cornea of the user's eye; and
Fig. 15 illustrates an example method.

### DETAILED DESCRIPTION

Examples of the disclosure relate to an apparatus 1 for monitoring a user's eye 11. The apparatus 1 uses an optical arrangement 5 comprising at least one light guide 13 and an exit pupil 15 to enable an image 31 to be projected onto the user's eye 11. Any distortion or other changes in the image 31 may be indicative of an irregularity in the user's eye 11. As the optical arrangement 5 comprises a light guide 13 and an exit pupil 15 this provides for a light weight apparatus 1 which could be integrated into a wearable device such as a near eye display. Also the use of the diffractive optical elements such as the light guide 13 means that there is no need to shine light directly into the user's eye 11. This provides for an apparatus 1 which is more convenient for a user 33 to use and may enable the user's eye 11 to be monitored over longer periods of time.

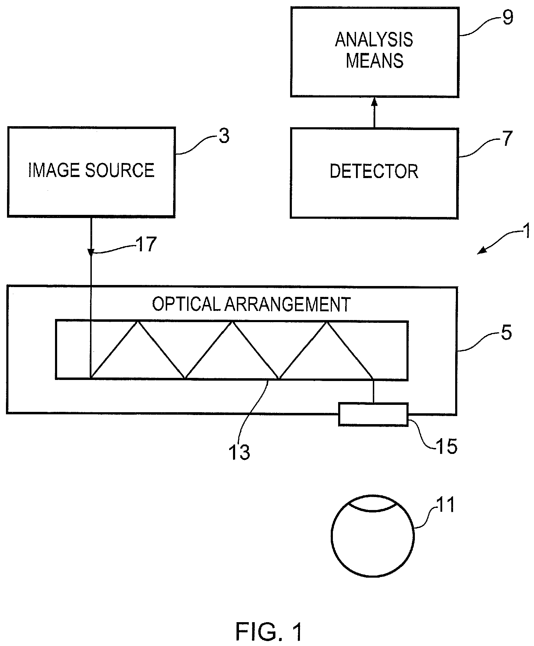

Fig. 1 schematically illustrates an apparatus 1 according to examples of the disclosure. The apparatus 1 comprises an image source 3, an optical arrangement 5, a detector 7 and analysis means 9 for enabling the detected image to be analysed. It is to be appreciated that the apparatus 1 may comprise additional components in some examples of the disclosure. For instance, the apparatus 1 could comprise additional optical elements which are not shown in Fig. 1.

The image source 3 may comprise any means for providing an image. In some examples the image source 3 comprises one or more light sources. The one or more light sources could comprise one or more light emitting diodes or any suitable light sources. The light source could be arranged to provide light of a single wavelength or a range of different wavelengths.

In some examples the image source 3 comprises a plurality of collimated light sources 34. The plurality of collimated light sources maybe arranged so as to produce a particular image. For example the plurality of collimated light sources may be arranged in a curved line or in any other suitable arrangement. An array of lenses 38 or other optical elements may be provided to focus the light from the collimated light sources.

In some examples the image source 3 may comprise means for providing structured light. The structured light may comprise a beam of light which comprises light and dark fringes. The light and dark fringes could comprise different intensities of light. The darker fringes may have a lower intensity compared to the light fringes so that less light is provided within the dark fringes than the light fringes. In some examples the dark fringes could be arranged so that none, or very few, of the photons from the beam of light fall within the dark fringes. In such examples the intensity of the light in the dark fringes could be zero or close to zero. The intensity of the light in the light fringes may be much higher than the intensity of light in the dark fringes. The light and dark fringes may be arranged in lines, such as parallel lines or in any other suitable pattern.

The means for providing the structured light could comprise a light source and a mask, or other optical element, which enables a pattern to be projected onto the beam of light provided by the light source. The pattern within the structured light will deform when it is incident on an object such as a user's eye 11. The deformation of the pattern can be used to determine the shape of the user's eye 11 or any other irregularities within the user's eye 11.

In some examples the image source 3 may comprise one or more displays 51. The displays 51 could be LED displays or any other suitable type of displays. The display 51 may be controlled by controlling circuitry. The controlling circuitry may enable different images to be displayed on the display 51. This may enable different images 31 to be projected onto the user's eye 11. This may enable different conditions of the user's eye 11 to be identified. For example, different images 31 may be arranged to make different types of deformations to be easier detect. This may enable different types of deformations, and the different conditions which cause the deformation to be detected. For instance, an abrasion of the cornea might be more easily detected with a coloured image 31 while a change in shape of the user's eye 11 could be detected with a black and white image 31.

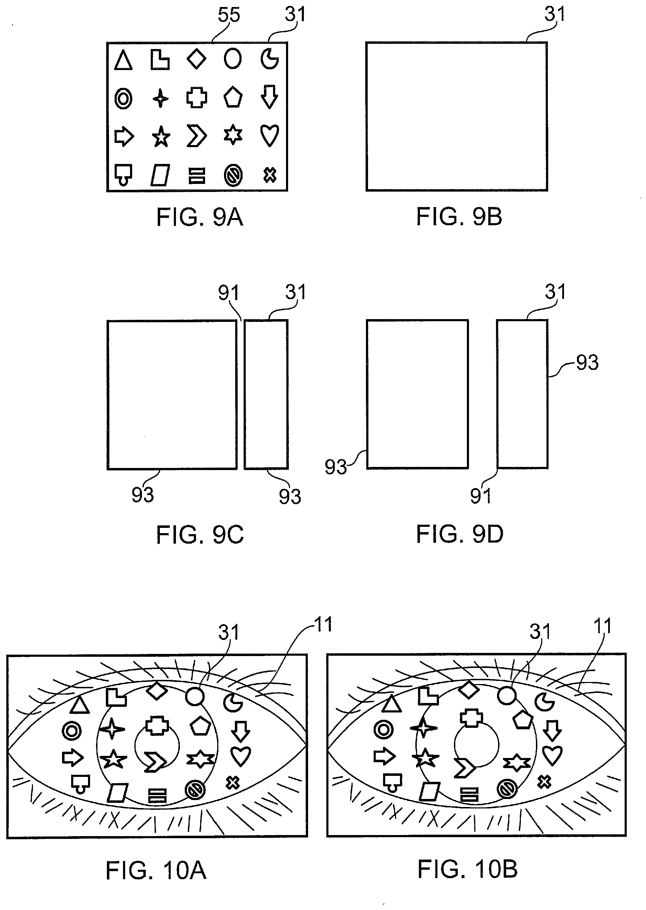

The image 31 provided by the image source 3 may comprise any suitable image which can be conveyed through the optical arrangement 5 and projected onto the user's eye 11. The image 31 may be arranged so that changes in the image 31 projected onto the user's eye 11 can be detected by one or more detectors 7. The changes in the image 31 could comprise a distortion of the image 31. This distortion could be detected as a change in position of one or more elements within the image 31 or a change in the separation between one or more elements within the image 31. In some examples the change in the image 31 could comprise a change in the colour of the projected image 31. For example, if there is an abrasion on the surface of the user's eye 11 this may change the colour of projected image 31 in the region of the abrasion. Example images 31 that could be used in examples of the disclosure are shown in Figs. 9A to 9D.

The optical arrangement 5 is coupled to the image source 3 so that a beam of light 17 provided by the image source 3 is coupled into the optical arrangement 5. The optical arrangement 5 may comprise any means for conveying a beam of light 17 from the image source 3 to the user's eye 11. The optical arrangement 5 therefore enables an image 31 from the image source 3 to be provided to the user's eye 11.

In examples of the disclosure the optical arrangement 5 may comprise one or more diffractive optical elements. The diffractive optical elements may be arranged to diffract the beam of light 17 from the image source 3 towards the user's eye 11.

The optical arrangement 5 may comprise at least one light guide 13 and an exit pupil 15. The optical arrangement 5 may be arranged within the apparatus 1 so that, when the apparatus 1 is in use, the exit pupil 15 is arranged to be positioned proximate to a user's eye 11. The light guide 13 is arranged to convey a beam of light 17 from the image source 3 to the exit pupil 15 to enable an image from the image source 3 to be projected onto the user's eye 11. The optical arrangement 5 may be controlled so that the image 31 is projected onto the cornea of the user's eye 11 or onto any other suitable part of the user's eye 11.

In some examples a plurality of light guides 13 may be provided within the optical arrangement 5. In some examples two or more light guides 13 may be arranged to enable images 31 from a single image source 3 to be provided to different eyes 11. In other examples a plurality of light guides 13 could be arranged to enable an image 31 from a single image source 3 to be provided to a single eye 11.

The optical arrangement 5 may be arranged so to increase the area of the user's eye 11 which is covered by the projected image 39. For example the optical arrangement 5 maybe arranged to expand or duplicate the image 31 to increase the area of the image before it is projected onto the user's eye 11.

The optical arrangement 5 may be arranged to project the image onto a particular part of the user's eye 11. For example, the optical arrangement maybe arranged to project the image 31 onto the cornea or other part of the user's eye 11. In some examples the apparatus 1 may comprise means for determining the position of the pupil, or other part of the user's eye 11 and means for aligning the exit pupil 15 with the pupil or other part of the user's eye 11. For example the apparatus 1 may comprise gaze tracking means which can detect the position of the pupil and the optical arrangement 5 may be adjustable so that the position of the exit pupil 15 can be aligned with the position of the pupil.

The detector 7 may comprise any means which may be arranged to detect the image 31 projected onto the user's eye 11. The detector 7 may be positioned within the apparatus 1 so that it can detect the images 31 projected onto the user's eye 11. In some examples the detector 7 may comprise an image 31 capturing device which is configured to capture an image of the user's eye 11 and the image projected onto the user's eye 11.

In some examples the detector 7 may comprise a detecting light source which may be arranged to illuminate the user's eye 11. The detecting light source may be provided in addition to a light source within the image source 3. The detecting light source may have a different wavelength of light to the light source 3 which provides the image 21. In some examples the detecting light source may use infrared light.

In some examples the apparatus 1 could be arranged so that the image detected by the detector 7 could be used to determine the position of the user's pupil or other part of the user's eye 11. In such examples the apparatus 1 may comprise image processing circuitry or any other suitable means which may be arranged to analyse the image and determine the position of the user's pupil or other part of the eye 11. Once the position of the pupil or other part of the eye 11 has been determined this information may be used to adjust the optical arrangement 5 so that the exit pupil 15 is aligned with the pupil, or other part of the user's eye 11.

The detector 7 is coupled to the analysis means 9 so that the images detected by the detector 7 are provided to the analysis means 9.

In some examples the analysis means 9 comprises image processing circuitry. The image processing circuitry may be provided within the apparatus 1. In some examples the analysis means 9 may comprise one or more transceivers arranged to transmit the detected image to a remote device to enable image processing circuitry within the remote device to analyse the image. In such examples the apparatus 1 might not comprise any image processing circuitry or might only comprise limited image processing circuitry.

The image processing circuitry, whether it is contained within the apparatus 1 or within a remote device, may be arranged to analyse the detected image to determine if there is any distortion or other types of changes between the image 31 projected onto the user's eye 11 and the image 31 detected by the detector 7. The distortion or other changes in the image 31 could be an indication that there is an irregularity in the user's eye 11. For example, a distortion of the image 31 could provide an indication that the shape of the user's eye 11 is irregular. A change in the colour of the image 31 could provide an indication that there is an abrasion or other irregularity within the user's eye 11. If the image processing circuitry determines that there is a change in the image 31 then information about the change could then be provided to the user 33 or to a medical practitioner to enable further tests on the condition of the user's eye 11 to be carried out.

In examples of the disclosure the apparatus 1 may enable the user's eye 11 to be monitored over periods of time. For example the apparatus 1 could be worn by the user 33 for a continuous period of time. In some examples the apparatus 1 could be used by the same user 33 at intervals over a period of time to enable changes in their eye 11 to be monitored. The intervals could be days, weeks or months so as to enable long term conditions of the user's eye 11 to be monitored. In such examples the images 31 that are detected by the detector 7 could be stored and used so that images 31 obtained at different times may be compared with each other.

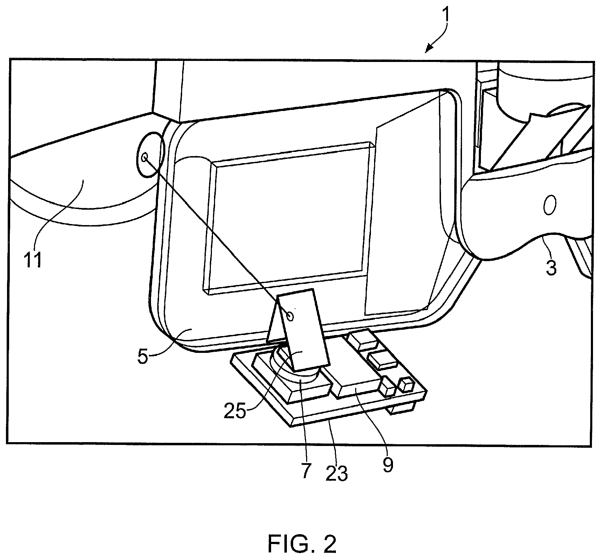

Fig. 2 illustrates an example apparatus 1 being used by a user 33. The example apparatus 1 comprises an image source 3, an optical arrangement 5, a detector 7 and analysis means 9.

In the example of Fig. 2 the apparatus 1 is provided within a wearable device 21 such as a head mounted display. The apparatus 1 is provided within the wearable device 21 so that the exit pupil 15 of the optical arrangement 5 is positioned adjacent to the user's eye 11 when the user 33 is wearing the wearable device 21.

In the example apparatus 1 of Fig. 2 the image source 3 is provided to the side of the user's eye 11. The image source 3 is positioned in the wearable device 21 so that it as adjacent to the user's nose. The light guides 13 within the optical arrangement 5 are arranged to convey the beam of light 17 provided by the image source 3 laterally toward the user's eye 11. This means that there is no need for a beam of light 17 to be shone directly into the user's eye 11.

The detector 7 is positioned within the wearable device 21 so that the detector 7 is positioned in front of the user's eye 11. One or more optical elements 25 may be provided within the apparatus 1 to direct images of the user's eye 11 onto to the detector 7. The detector 7 may be positioned on the opposite side of the optical arrangement 5 so that the images captured by the detector 7 pass through one or more elements of the optical arrangement 5 before they are detected by the detector 7.

In the example of Fig. 2 the detector 7 comprises an image capturing device. The image capturing device may be an infrared camera or any other suitable type of camera. The image capturing device may comprise a CMOS (complementary metal-oxide-semiconductor) sensor, a CCD (charge coupled device) sensor, or any other suitable type of sensor.

In the example of Fig. 2 the detector 7 is mounted on a circuit board 23. Other components of the apparatus 1 may also be mounted on the same circuit board 23. For example, the analysis means 9 may also be provided on the circuit board 23. The analysis means 9 could comprise image processing circuitry and/or one or more transceivers as described in this description.

In the example of Fig. 2 the apparatus 1 is provided within the wearable device 21. In other examples other means could be used to attach the apparatus 1 to a wearable device 21. For example the apparatus 1 could be a separate attachment which could be attached to a user's glasses or sunglasses. In some examples the apparatus 1 could be a separate attachment which could be attached to a virtual or augmented reality headset or other head mounted display.

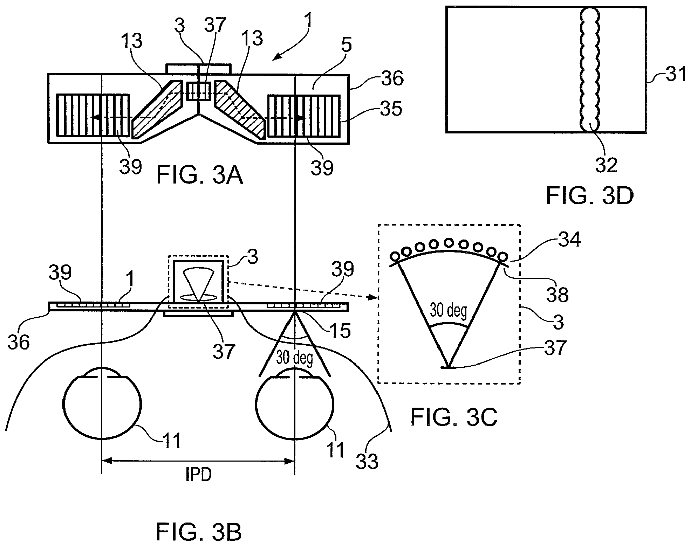

Figs. 3A to 3D illustrate an example apparatus 1 and projected image 31 that may be used in some examples of the disclosure. Fig. 3A shows a front view of the apparatus 1, Fig. 3B shows a plan view of the apparatus 1 being worn by a user 33, Fig. 3C shows the image source 3 in more detail and Fig. 3D shows the image 31 projected by the image source 3.

In the examples of Figs. 3A to 3D the apparatus 1 is provided within a wearable device 21. The wearable device 21 is configured to be worn on the head of the user 33. In the example of Figs. 3A to 3D the wearable device 21 comprises a head mounted display which enables the exit pupil 15 of the optical arrangement 5 to be positioned adjacent to the user's eye 11. Other types of wearable device 21 which enable the exit pupil 15 to be positioned adjacent to the user's eye 11 may be used in other examples of the disclosure.

In Fig. 3B the apparatus 1 is illustrated positioned adjacent to the head of the user 33 as it may be arranged during use. The cross section of the face of the user 33 is illustrated to provide an indication of how the apparatus 1 may be positioned in use.

In the examples of Figs. 3A to 3D the light guides 13 within the optical arrangement 5 comprise an exit pupil expander 35. The exit pupil expander 35 may comprise means for increasing the size of the exit pupil 15 of the optical arrangement 5. The exit pupil expander 35 may be configured to increase the size of the exit pupil 15 of the optical arrangement 5 so that the exit pupil 15 of the optical arrangement 5 may be larger than the exit pupil of the image source 3. This may increase the size of the image before it is projected onto the user's eye 11. It is to be appreciated that other types of light guide elements 13 could be used in other examples of the disclosure.

The exit pupil expander 35 may comprise one or more substrates 36. The substrates 36 of the exit pupil expander 35 comprise a first diffraction grating 37 and a second diffraction grating 39. The diffraction gratings 37, 39 may comprise any suitable periodic structure.

The first diffraction grating 37 may be an in-coupling grating which couples an incident beam of light 17 into the substrate 35. The incident beam of light 17 may be provided by the image source 3. The second diffraction grating 39 may be an out-coupling grating which couples the beam of light out of the substrate 36. The second diffraction grating 39 is positioned so that the out-coupled beam of light is incident on the user's eye 11. This enables the image provided by the image source 3 to be projected onto the user's eye 11.

The first diffraction grating 37 and the second diffraction grating 39 may have different sizes. In some examples the second diffraction grating 39 may be larger than the first diffraction grating 37 such that it covers a larger area of the surface of the substrate 35. This may cause expansion of the exit pupil of the beam of light 17. The sizes and period of the diffraction gratings 37, 39 may be selected to enable a focused image to be projected onto the user's eye 11.

The thickness of the substrate 36 which is used may be dependent upon the width of the diffraction gratings 37, 39 which are used, the wavelength of the light provided by the image source 3, the proximity of the light guide 13 to the user's eye 11 and any other suitable factor. The thickness of the substrate 36 that is used in the light guide 13 may be thinner than the thickness of the substrates used for exit pupil expanders in near eye displays which may be used for virtual or augmented reality applications. This may increase the number of internal reflections of the beam of light 17 within the substrate 36 which may decrease the quality of the image that is projected onto the user's eye 11. However the image quality required for monitoring the user's eye 11 may be lower than the image quality required for augmented or virtual reality applications. This may enable a thinner and/or more light weight apparatus 1 to be used to monitor the user's eyes 11.

In the example embodiment of Figs. 3A to 3D the first diffraction grating 37 is arranged so that a beam of light 17 from the image source 3 is incident on the first diffraction grating 37. The first diffraction grating 37 couples the incident beam of light 17 into the substrate 36. The in-coupled beam of light propagates through the substrate 36. The exit pupil expander 35 is configured to enable total internal reflection of the in-coupled beam of light. This means that the in-coupled beam of light remains inside the substrate 36 until it is incident upon the second diffraction grating 39.

The second diffraction grating 39 may be configured to diffract the in-coupled beam of light out of the substrate 36 to provide an out-coupled beam of light. As the light guide 13 comprises an exit pupil expander 35 the out-coupled beam of light may have a larger exit pupil 15 than the beam of light 17 which is provided by the image source 3.

The image source 3 is positioned within the apparatus 1 so that, when the user 33 is wearing the apparatus 1 the image source 3 is positioned to the side of the user's eye 11. This means that any light provided by the image source 3 is not shone directly into the user's eye 11. In the examples of Figs. 3A to 3D the image source 3 is positioned in the center of the apparatus 1. In the example of Figs. 3A to 3D the image source 3 is positioned within the apparatus 1 so that when the user 33 is wearing the apparatus 1 the image source 3 is positioned over the user's nose or adjacent to the user's nose.

In the example of Figs. 3A to 3D the image source 3 comprises a plurality of collimated light sources 34. In the example of Fig. 3A each of the light sources 34 is identical so that each of the light sources 34 are the same size and provide light the same wavelength and the same intensity.

In the example of Figs. 3A to 3D the image source 3 also comprises a plurality of lenses 38 positioned between the light sources 34 and the first diffraction grating 37 of the optical arrangement 5. The lenses 38 may be arranged to focus the light from the plurality of collimated light sources 34 onto the first diffraction grating 37 so that the light from the light sources 34 is coupled into the exit pupil expander 35.

In the examples of Figs. 3A to 3D the plurality of light sources 34 are provided in a curved array so as to provide a curved image. The curvature of the array may correspond to the curvature of the user's eye 11 so that when the image 31 is projected onto the user's eye 11 any irregularities in the shape of the user's eye 11 may be detected.

In the example of Figs. 3A to 3D the curved array comprises an arc which spans an angle of 30 degrees. The curved array could span a different angle in other examples of the disclosure.

In the example of Figs. 3A to 3D the collimated light sources 34 are provided in a line so that the image 31 provided by the image source 3 comprises a single bright line 32 corresponding to the plurality of light sources 34 as shown in Fig. 3D. This image could be used to detect any changes in the shape of the user's eye 11. For example, if the user's eye 11 has an irregular shape this would distort the line 32 when the image 31 is projected onto the user's eye 11. This distortion could be detected by the detector 7 and the analysis means 9 which are not shown in Figs. 3A to 3D.

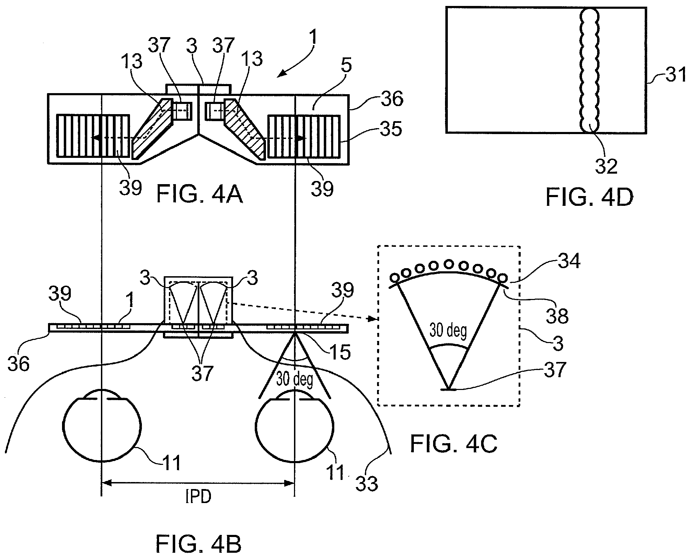

In the example apparatus of Figs. 3A to 3D the apparatus 1 comprises a single image source 3. The optical arrangement 5 is arranged so that the image 31 provided by the image source 3 is conveyed to both of the user's eyes 11. In this example the image 31 that is provided to each of the eyes 11 may be identical. In other examples of the disclosure more than one image source 3 could be provided. Figs. 4A and 4D illustrate another example embodiment in which two image sources 3 are provided. Fig. 4A shows a front view of the apparatus 1, Fig. 4B shows a plan view of the apparatus 1 being worn by a user 33, Fig. 4C shows one of the image sources 3 in more detail and Fig. 4D shows an image 31 projected by one of the image sources 3.

The apparatus 1 in the examples of Figs. 4A to 4D is similar to the apparatus 1 shown in the examples of Figs. 3A to 3D and corresponding reference numerals are used for corresponding components. The apparatus 1 in the examples of Figs. 4A to 4D differs from that of the examples of Figs. 3A to 3D because in the example of Figs. 4A to 4D the apparatus 1 comprises two image sources 3 whereas in the example of Figs. 3A to 3D the apparatus 1 only comprises a single image source 3. Also in the example of Figs. 4A to 4D the optical arrangement 5 comprises two first diffraction gratings 37 so that a first diffraction grating 37 is provided for each of the image sources 3. Each of the first diffraction gratings 37 are positioned within the apparatus 1 so that a beam of light 17 from an image source 3 is incident on the first diffraction grating 37.

In the example of Figs. 4A to 4D both of the image sources 3 comprises a plurality of collimated light sources 34. The plurality of collimated light sources 34 may be as shown in Figs. 3A to 3D to provide an image 31 comprising a single line 32. In the example of Figs. 4A to 4D the two image sources 3 are identical so that two identical images 31 are provided. In other examples the image sources 3 could be different so that different images can be provided to each of the user's eyes 11.

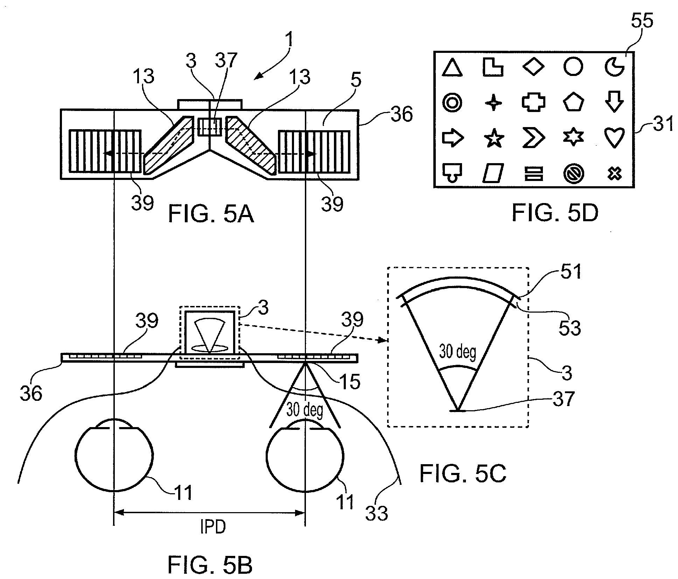

Figs. 5A to 5D illustrate another example embodiment in which a different type of image source 3 is used. Fig. 5A shows a front view of the apparatus 1, Fig. 5B shows a plan view of the apparatus 1 being worn by a user 33, Fig. 5C shows the image sources 3 in more detail and Fig. 5D shows an example image 31 which could be projected by the image sources 3.

The optical arrangement 5 in the examples of Figs. 5A to 5D is similar to the optical arrangement 5 shown in the examples of Figs. 3A to 3D and corresponding reference numerals are used for corresponding features. The apparatus 1 in the examples of Figs. 5A to 5D differs from that of the examples of Figs. 3A to 3D because in the example of Figs. 5A to 5D the image source 3 comprises a display 51 rather than the plurality of collimated light sources 34.

The display 51 used in the image source 3 may be any suitable type of display such as an OLED (organic light emitting diode) display. The display 51 may be a curved display so as to provide a curved image. The curvature of the display 51 may correspond to the curvature of the user's eye 11 so that when the curved image generated by the display 51 is projected onto the user's eye 11 any irregularities in the shape of the user's eye 11 may be detected.

In the example of Figs. 5A to 5D the curved display 51 comprises an arc which spans an angle of 30 degrees. The curved display 51 could span a different angle in other examples of the disclosure.

The image source 3 also comprises an array of collimating lenses 53. The collimating lenses 53 are provided overlaying the display 51 and arranged to focus the image 31 from the display 51 onto the first diffraction grating 37 of the optical arrangement 5.

Fig. 5D illustrates an example image 31 that may be provided by the display 51. The example image comprises an array 55 of objects. The objects may comprise two dimensional shapes. Each of the objects comprises a different two dimensional shape in the example of Fig. 5D. In other examples two or more of the same shape could be repeated within the array 55. In examples of Fig. 5D each of the objects is a single colour. In other examples the objects could be multicolored.

The array 55 may be arranged so that when the image 31 is projected onto the user's eye 11 the spacing between the objects within the array 55 may be used to determine if there has been any distortion in the image 31 as detected by the detector 7. In the example of Fig. 5D the array 55 comprises a plurality of objects arranged in a plurality of columns and rows. In the example of Fig. 5D the columns are perpendicular to the rows. Other arrangements for the array 55 may be used in other examples of the disclosure. The arrangement of the objects within the array 55 may depend on the optical arrangement 5 or any other suitable factors.

In the example array 55 in Fig. 5D each of the objects are different. That is, there is no repeated object within the image 31. In other examples some of the objects may be replicated at different positions within the array 55.

The image 31 may be provided in two colours so that the objects are provided in a first colour and the background may be provided in a second colour. For examples the objects may be provided as white objects on a black background. In other examples the images 31 provided by the display 51 may be multi colour.

The display 51 may be arranged so that the image 31 provided by the display 51 can be changed. For example, this may enable different arrays 55 to be used at different times. Alternatively the display 51 could be used to display an image 31 that is not an array. Other types of images are shown in Figs. 9A to 9D.

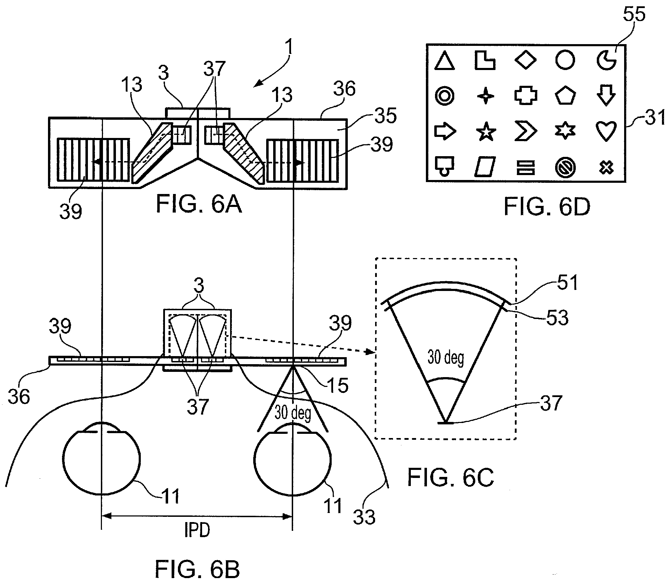

Figs. 6A to 6D illustrate another example embodiment in which two image sources 3, each comprising a display 51, are provided. Fig. 6A shows a front view of the apparatus 1, Fig. 6B shows a plan view of the apparatus 1 being worn by a user 33, Fig. 6C shows one of the image sources 3 in more detail and Fig. 6D shows an image 31 projected by one of the image sources 3.

The apparatus 1 in the examples of Figs. 6A to 6D is similar to the apparatus 1 shown in the examples of Figs. 5A to 5D and corresponding reference numerals are used for corresponding components. The apparatus 1 in the examples of Figs. 6A to 6D differs from that of the examples of Figs. 5A to 5D because in the example of Figs. 6A to 6D the apparatus 1 comprises two image sources 3 whereas in the example of Figs. 5A to 5D the apparatus 1 only comprises a single image source 1. Also in the example of Figs. 6A to 6D the optical arrangement 5 comprises two first diffraction gratings 37 so that a first diffraction grating 37 is provided for each of the image sources 3. Each of the first diffraction gratings 37 are positioned within the apparatus 1 so that a beam of light 17 from an image source 3 is incident on the first diffraction grating 37.

In the example of Figs. 6A to 6D both of the image sources 3 comprises a curved display 51. The curved displays 51 may be arranged as shown in Figs. 5A to 5D. Each of the curved displays 51 may provide an image 31 comprising an array 55.

In the example of Figs. 6A to 6D the two displays 51 are controlled so that two identical images 31 are provided. In other examples the displays 51 could be controlled so that different images 31 are provided by each of the displays 51. This enables different images 31 to be provided to each of the user's eyes 11.

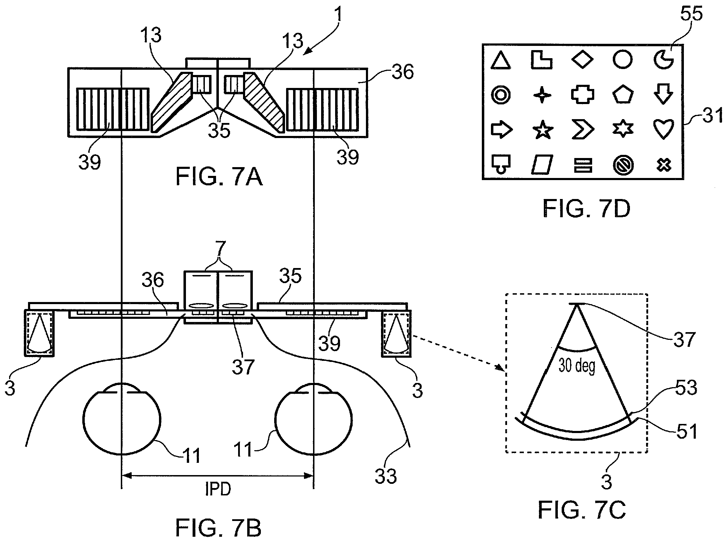

In each of the examples in Figs. 3A to 6D the image sources 3 are provided in the centre of the apparatus 1 so that when the apparatus 1 is worn by the user 33 the image sources 3 are positioned over the nose of the user 33. Figs. 7A to 8D illustrate examples in which the image sources 3 are provided in a different position within the apparatus 1.

Figs. 7A to 7D illustrate an example embodiment in which two image sources 3 comprising a display 51 are provided. Fig. 7A shows a front view of the apparatus 1, Fig. 7B shows a plan view of the apparatus 1 being worn by a user 33, Fig. 7C shows one of the image sources 3 in more detail and Fig. 7D shows an image 31 projected by one of the image sources 3.

The apparatus 1 in the examples of Figs. 7A to 7D is similar to the apparatus 1 shown in the examples of Figs. 6A to 6D and corresponding reference numerals are used for corresponding components. The apparatus 1 in the examples of Figs. 7A to 7D differs from that of the examples of Figs. 6A to 6D because in the example of Figs. 7A to 7D the image sources 3 are provided at the outer edges of the apparatus 1 rather than in the center. The image sources 3 are positioned within the apparatus 1 so that when the user 33 is wearing the apparatus 1 the image sources 3 are provided towards the edge of the user's face.

The optical arrangement 5 is also arranged so that the first diffraction gratings 37 are provided towards the outer edges of the apparatus 1 rather than towards the centre to ensure that the light beams 17 from the image sources 3 are coupled into the exit pupil expanders 35.

In the example of Figs. 7B the detectors 7 are shown. The detectors 7 may be arranged to detect the images that are projected onto the user's eye 11. The detectors 7 may be positioned towards the centre of the apparatus 1 or in any other suitable locations.

In the example of Figs. 7A to 7D the image sources 3 comprise displays 51 and the example image 31 is the same as the images provided in Figs. 5D and 6D. It is to be appreciated that the displays 51 could be arranged to provide different images in other examples of the disclosure.

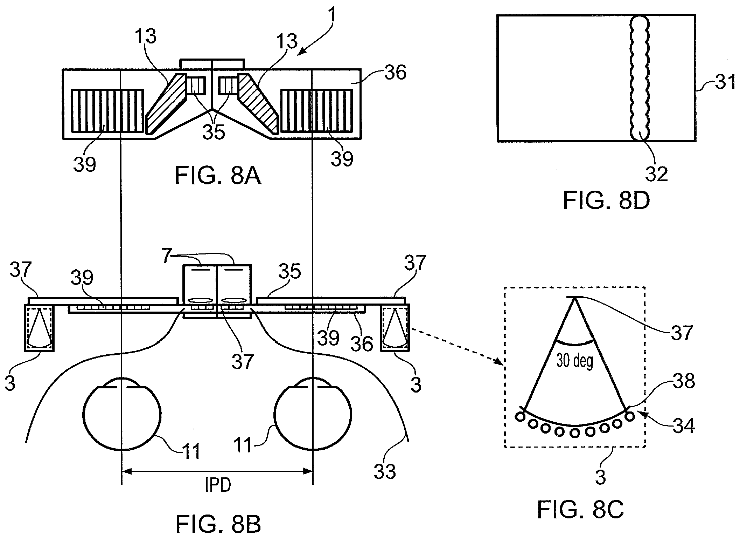

It is also to be appreciated that other types of image source 3 could be used in other examples of the disclosure. Figs. 8A to 8D illustrate another example embodiment in which a different type of image source 3 is used. Fig. 8A shows a front view of the apparatus 1, Fig. 8B shows a plan view of the apparatus 1 being worn by a user 33, Fig. 8C shows the image sources 3 in more detail and Fig. 8D shows an example image 31 which could be projected by the image sources 3.

The optical arrangement 5 in the examples of Figs. 8A to 8D is similar to the optical arrangement 5 shown in the examples of Figs. 7A to 7D and corresponding reference numerals are used for corresponding features. The apparatus 1 in the examples of Figs. 8A to 8D differs from that of the examples of Figs. 7A to 7D because in the example of Figs. 8A to 8D the image source 3 comprises a plurality of collimated light sources 34 rather than a display 51. The plurality of collimated light sources 34 may be as described above. Other types of image sources 3 may be used in other examples of the disclosure.

Figs. 9A to 9D illustrate example projected images 31 that could be used in some examples of the disclosure. The example projected images 31 could be provided by a display 51 or by any other suitable image source 3.

The example image 31 in Fig. 9A comprises an array 55 of objects. The array 55 may be arranged so that when the image 31 is projected onto the user's eye 11 the spacing between the objects within the array 55 as detected by the detector 7 may be compared to the spacing in the projected image 31 and used to determine if there has been any distortion in the image 31. In the example of Fig. 9A the array 55 comprises a plurality of objects arranged in a plurality of columns and rows. In the example of Fig. 9A the columns are perpendicular to the rows. In the example of Fig. 9A the objects within the array 55 have a regular spacing so that the spacing between adjacent objects in a row is the same and the spacing between adjacent objects in a column is the same. Other arrangements for the array 55 may be used in other examples of the disclosure. The arrangement of the objects within the array 55 may depend on the optical arrangement 5 of the apparatus 1 or any other suitable factors.

The example image 31 in Fig. 9B shows a block of uniform colour. The colour could be any suitable colour. In some examples the colour could be blue. The blue block could be provided by a display 51, or by a blue light source or by any other suitable means.

The example image 31 in Fig. 9C comprises structured light. The image 31 provided by the structured light comprises a light fringe 91 and two dark fringes 93. The light fringe 91 provides a straight vertical line within the image 31. The straight vertical line is provided towards the right hand side of the image 31. Other arrangements for structured light may be used in other examples of the disclosure. For example a plurality of lines may be used in some examples.

The structured light may be provided by any suitable type of image source 3. In some examples the structured light could be provided by a light source and a mask positioned in front of the light source. In other examples the structured light could be provided by a display 51. Where the structured light is provided by a display 51 this may enable the display 51 to be controlled to change the image 31 provided by the structure light.

Fig. 9D shows another example image 31 provided by structured light. In the example of Fig. 9D the image also comprises a light fringe 91 and two dark fringes 93. However, in the example of Fig. 9D the light fringe 91 is thicker than in the example of Fig. 9C. The thickness of the light fringe 91 could be controlled by controlling a display 51 or by using a different mask with a light source or by controlling the position of the mask relative to the light source or by any other suitable means.

Figs. 10A and 10B illustrate example images 31 projected onto a user's eye 11. In the examples of Figs. 10A and 10B the image comprises an array 55 as shown in Fig. 9A in which the objects have a regular spacing.

Fig. 10A shows the image 31 projected onto an eye 11 which has no irregularity. The spacing between the objects in the image 31 remains regular. The spacing between the objects can be detected by the detector 7 and the analysis means 9.

Fig. 10B shows the image 31 projected onto an eye 11 which has an irregularity. In the example of Fig. 10B the irregularity is a change in shape. The change in shape could be a change in the curvature of the cornea. This change in the shape of the eye 11 distorts the image 31 so that the spacing between the objects in the array 55 becomes irregular. That is, there is a larger spacing between adjacent objects in some rows and columns than there are in other rows and columns. The change in the spacing between the objects can be detected by the detector 7 and the analysis means 9.

It is to be appreciated that other changes in the image 31 could be detected in other examples of the disclosure. For example, a change in colour of the image 31 could be indicative of an abrasion or other type of irregularity within the eye 11. In such examples the image 31 that is used could be a single block of colour as shown in Fig. 9B.

If a change in shape, or other irregularity is detected then this information could be provided to the user 33, or to a medical professional to indicate that an irregularity has been detected. This could prompt further examination and/or tests of the user's eye 11.

In examples of the disclosure it may be useful to enable the image 31 to be projected onto as large a surface of the user's eye 11 as possible. For example, if the whole of the cornea can be covered by the image 31 then this may enable the whole of the cornea to be monitored.

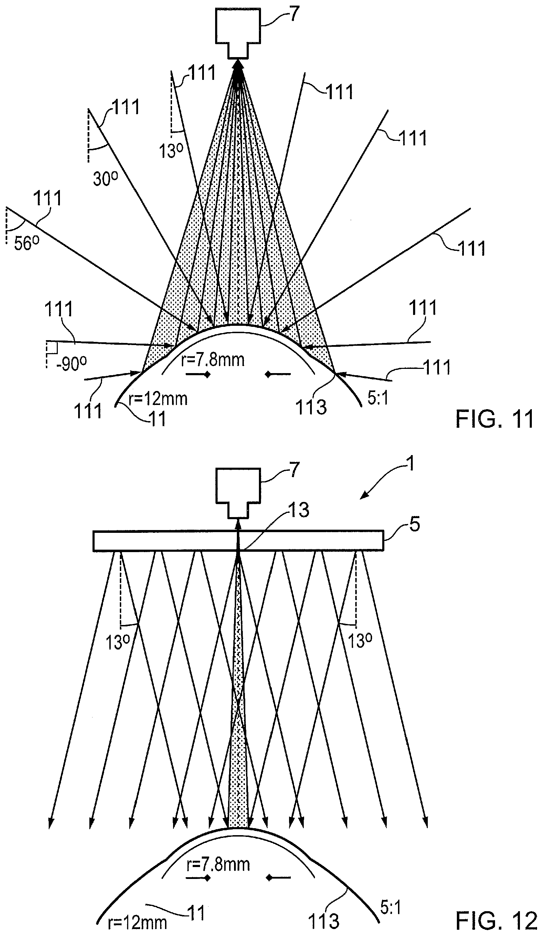

Fig. 11 schematically illustrates incoming light beams 111 required for full coverage of the user's cornea 113. This shows that the incoming light beams 111 which provide the projected image 31 would need to come from a range of angles extending more than 90 degrees from the perpendicular on either side. The entire of the cornea 113 may be detected by the detector 7.

Fig. 12 schematically illustrates the coverage of the user's cornea 113 that may be obtained with an apparatus 1 in some examples of the disclosure. This shows that where the image source 3 and optical arrangement 5 provide an output with a narrow angular range, only a portion of the user's cornea 113 can be covered by the image 31.

In the example of Fig. 12 the optical arrangement 5 provides an output beam of light with an angular coverage of 13 degrees either side of the perpendicular. It can be seen in Fig. 12 that this only covers a small portion of the cornea 113 of the user's eye 11. The portion of the cornea 113 that is covered in the example of Fig. 12 may be smaller than the pupil of the eye 11.

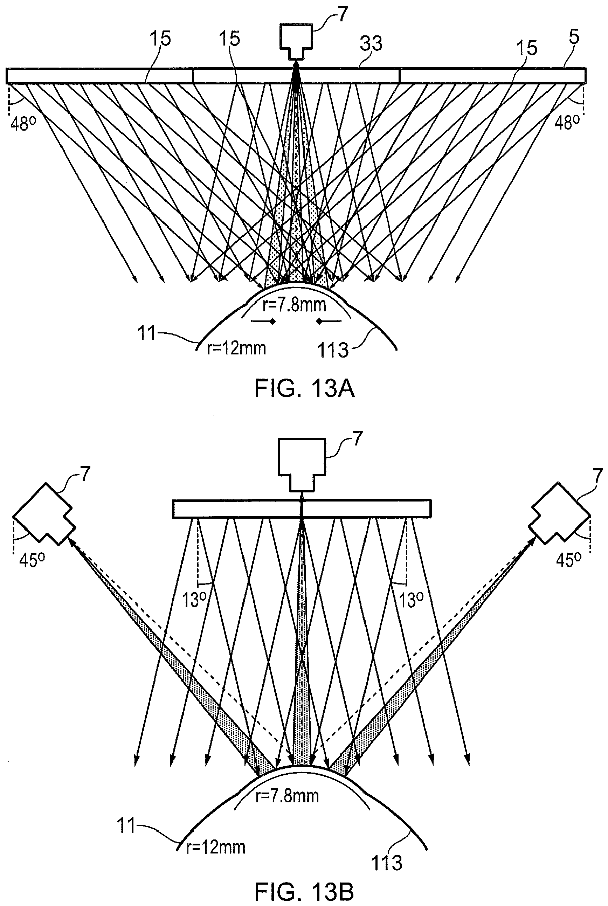

In some examples it may be sufficient for the image 31 provided by the image source 3 and optical arrangement 5 to only cover a small portion of the cornea 113 of the user's eye 11. For example the apparatus 1 may be arrange to adjust the optical arrangement 5 so that the exit pupil 15 of the optical arrangement 5 is aligned with a particular part of the user's eye 11 which is of interest to the user 33 or a medical professional. In other examples the apparatus 1 may be arranged to increase the coverage of the cornea 113. Figs. 13A to 13C illustrate example apparatus 1 arranged to obtain extended coverage of the cornea 113 of the user's eye 11.

In the example of Fig. 13A the coverage of the cornea 113 is increased by increasing the number of outputs provided by the optical arrangement 5. In such examples the optical arrangement 5 may be arranged to comprise a plurality of exit pupils 15 and outputs maybe provided by each of the exit pupils 15, or a subset of the exit pupils 15. This therefore increases the overall size of the image 31 provided by the apparatus 1. In some examples the outputs provided by the exit pupils 15 may be provided at different angles to ensure that the curved surface of the cornea 113 is covered by the image 31.

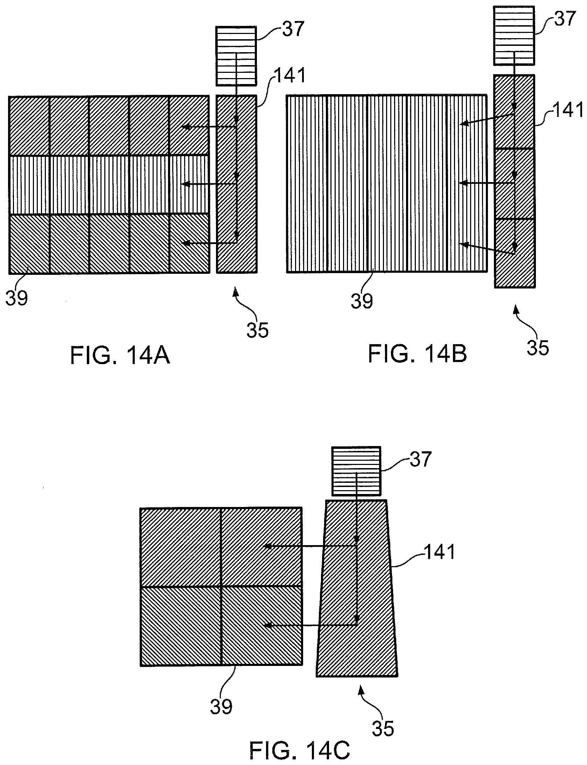

In examples where the optical arrangement 5 comprises an exit pupil expander 35 the exit pupil expander 35 may comprise a plurality of second diffraction gratings 39 to provide the additional exit pupils 15. The second diffraction gratings 39 are arranged to out-couple a beam of light from the substrate 36. In such examples each of the second diffraction gratings 39 may provide an image so that the image 31 projected onto the user's eye 11 is a composite image comprised of the plurality of images from each of the second diffraction gratings 39. In such examples the composite image may comprise the same image duplicated a plurality of times. Figs. 14A to 14C illustrate example arrangements of second diffraction gratings 39 that may be used to provide a composite image in some examples of the disclosure.

In the example of Fig. 13B the coverage of the cornea 113 is increased by providing a plurality of detectors 7 at a plurality of different angles relative to the user's eye 11. In the example of Fig. 13B a first detector 7 is provided straight in front of the user's eye 11, a second detector 7 is provided offset at 45 degrees to the right and a third detector 7 is provided offset at 45 degrees to the left. This enables images from covering different sections of the cornea 113 to be detected by the detector 7.

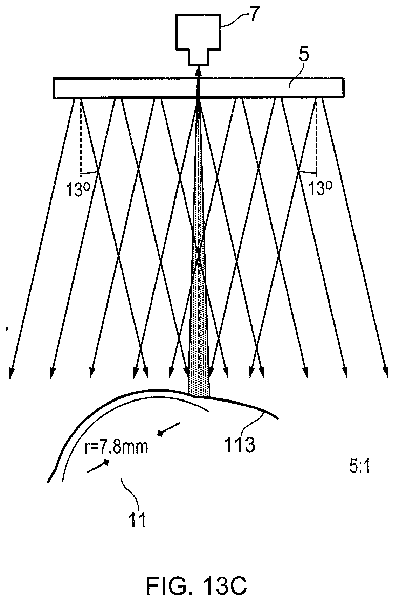

In the example of Fig. 13C the coverage of the cornea 113 is increased by moving the eye 11 relative to the apparatus 1. This could be achieved by instructing the user 33 to move their eye 11 as required or by tracking the movement of the user's eye 11 while they are using the apparatus 1. In other examples the movement could be achieved by moving the apparatus 1 or portions of the apparatus 1 relative to the users' eye 11. For example, in some embodiments, the optical arrangement 5 may be arranged to enable the exit pupil 15 to be moved relative to the user's eye 11.

It is to be appreciated that other methods of obtaining the larger coverage could be used in other examples of the disclosure. For example, the optical arrangement 5 could be arranged to give an output with a larger angle of coverage. This could be achieved by using material with a high refractive index for the substrate 36 of the exit pupil expander 35 so as to achieve larger angles for the total internal reflection.

Figs. 14A to 14C show diffraction gratings that may be arranged within example exit pupil expanders 35 to obtain extended coverage of the user's cornea 113.

In the example of Fig. 14A a first diffraction grating 37 is provided. The first diffraction grating 37 may be an in-coupling diffraction grating arranged to in-couple a beam of light 17 from the image source 3. In the example of Fig. 14A the exit pupil expander 35 also comprises an intermediate diffraction grating 141 which is arranged to diffract the in-coupled beam of light towards the second diffraction grating 39.

The second diffraction grating 39 provides the out-coupling grating and is arranged to out-couple the beam of light from the exit pupil expander 35. In the example of Fig. 14A the second diffraction grating 39 is divided into a plurality of regions so that each region out-couples the beam of light from the exit pupil expander 35. The different regions of the second diffraction grating 39 may have different specifications for the grating. For example the angle of the gratings may be different and/or the spacing of the grating may be different.

In the example of Fig. 14A each region the second diffraction grating 39 out-couples the same image or a similar image. This creates a larger out-coupled image. The larger out-coupled image is a composite image comprising a plurality of smaller images. The composite image may comprise duplications of the smaller image. The larger image provides for increased coverage of the cornea 113.

In the example of Fig. 14A the second-diffraction grating 39 is divided into fifteen different regions to provide the composite image output. It is to be appreciated that other arrangements could be used in other examples of the disclosure.

Fig. 14B illustrates another example in which the second diffraction grating 39 also provides a composite image. The example exit pupil expander 35 of Fig. 14B also comprises a first diffraction grating 37, a second diffraction grating 39 and an intermediate diffraction grating 141. In the example of Fig. 14B the intermediate diffraction grating 141 comprises different regions which diffract the beams of light at different angles. This helps to increase the number of images provided by the second-diffraction grating 39. In the example of Fig. 14B the intermediate diffraction grating 141 comprises three different regions. The grating has different angular arrangement in the different regions.

In the example of Fig. 14B the second diffraction grating 39 only has five different regions. However, as the intermediate diffraction grating 141 has a plurality of different regions this still provides for a composite image comprising a plurality of output images.

Fig. 14C illustrates another example in which the second diffraction grating 39 also provides a composite image. The example exit pupil expander 35 of Fig. 14C also comprises a first diffraction grating 37, a second diffraction grating 39 and an intermediate diffraction grating 141. In the example of Fig. 14C the second diffraction grating 37 comprises four different regions arranged in a square arrangement. Other types of arrangement may be used in other examples of the disclosure.

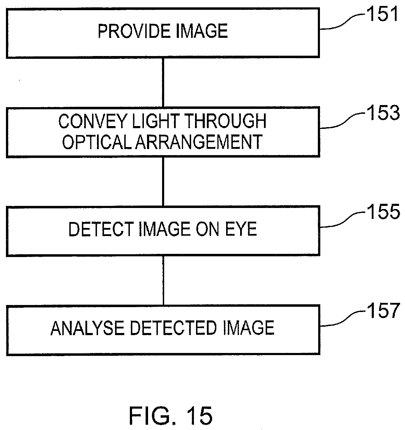

Fig. 15 illustrates an example method that may be implemented in some examples of the disclosure. The method may be implemented using example apparatus 1 as described in this application.

At block 151 the method comprises providing an image 31 from an image source 3. At block 153 the method comprises conveying a beam of light 17 through an optical arrangement 5 to enable an image 31 from the image source 3 to be projected onto a user's eye 11 wherein the optical arrangement 5 comprises at least one light guide 13 and an exit pupil 15 where the exit pupil 15 is arranged to be positioned proximate to the user's eye 11 and the at least one light guide 13 is arranged to convey a beam of light 17 from the image source 3 to the exit pupil 15.

The method also comprises, at block 155, detecting the image 31 projected onto the user's eye 11 and, at block 157, enabling the detected image to be analysed so as to identify changes in the projected image 31.

It is to be appreciated that the method may comprise additional blocks that are not illustrated in Fig. 15. For instance, in some examples the method may comprise arranging the apparatus so that the exit pupil 15 of the optical arrangement 5 is aligned with the user's eye 11 or with a particular part of the user's eye 11. This may enable the image 31 to be projected onto a selected part of the user's eye 11.

Examples of the disclosure provide for an apparatus 1 and method of monitoring a user's eye 11. The apparatus 1 uses a diffractive optical arrangement which does not shine light directly into a user's eye 11. This may make the apparatus 1 more comfortable for the user 33 to use.

In some examples the apparatus 1 may be integrated into a wearable device 21. For example, the apparatus 1 could be integrated into a near eye display. This wearable device 21 could be used for other uses such as augmented reality or virtual reality applications. This could make the apparatus 1 more convenient for the user 33 to use and may enable the monitoring to take place over extended periods of time.

The analysis of the image 31 projected on to the user's eye 11 can be performed by the detector 7 and the analysis means 9. This may provide for digital analysis of the image 31 which could be performed by the apparatus 1 or by a remote device. The information could then be provided to a medical professional if needed. This therefore may enable the monitoring of the user's eye 11 to be performed without any medical professional or any special training.

In this description the term coupled means operationally coupled. It is to be appreciated that any number of components may be provided between coupled elements, including zero components.

The term "comprise" is used in this document with an inclusive not an exclusive meaning. That is any reference to X comprising Y indicates that X may comprise only one Y or may comprise more than one Y. If it is intended to use "comprise" with an exclusive meaning then it will be made clear in the context by referring to "comprising only one..." or by using "consisting".

In this brief description, reference has been made to various examples. The description of features or functions in relation to an example indicates that those features or functions are present in that example. The use of the term "example" or "for example" or "may" in the text denotes, whether explicitly stated or not, that such features or functions are present in at least the described example, whether described as an example or not, and that they can be, but are not necessarily, present in some of or all other examples. Thus "example", "for example" or "may" refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. An apparatus comprising:
an image source;
an optical arrangement comprising at least one light guide and an exit pupil where the exit pupil is arranged to be positioned proximate to an eye of a user and the at least one light guide is arranged to convey a beam of light from the image source to the exit pupil to enable an image from the image source to be projected onto the user's eye;
a detector arranged to detect the image projected onto the user's eye; and
means for enabling the detected image to be analysed so as to identify changes in the projected image.

2. An apparatus as claimed in claim 1 wherein the means for enabling the detected image to be analysed comprises image processing circuitry.

3. An apparatus as claimed in any preceding claim wherein the means for enabling the detected image to be analysed comprises one or more transceivers arranged to transmit the detected image to a remote device to enable the remote device to analyse the image.

4. An apparatus as claimed in any preceding claim wherein the apparatus is provided in a wearable device.

5. An apparatus as claimed in any preceding claim wherein the image source comprises at least one of; a light source, a plurality of collimated light sources, at least one display, at least one curved display.

6. An apparatus as claimed in any preceding claim wherein the image provided by the image source comprises an array of objects.

7. An apparatus as claimed in any preceding claim wherein the light guide comprises one or more diffraction gratings.

8. An apparatus as claimed in claim 7 wherein the light guide and the one or more diffraction gratings provide an exit pupil expander.

9. An apparatus as claimed in any preceding claim wherein the optical arrangement is arranged to duplicate the image provided by the image source so that a plurality of duplicated images are projected onto the user's eye.

10. An apparatus as claimed in any preceding claim where the diffraction gratings in the light guide element are arranged to cause duplication of the image.

11. An apparatus as claimed in any preceding claim wherein the detector comprises an image capturing device configured to capture an image of the user's eye and the image projected onto the user's eye.

12. An apparatus as claimed in claim 11 wherein the detector comprises a light source configured to illuminate the user's eye using infrared light.

13. An apparatus as claimed in any of claims 11 to 12 wherein the apparatus comprises means for using the image detected by the detector to determine the position of the user's pupil.

14. An apparatus as claimed in any preceding claim wherein the optical arrangement comprises adjustment means arranged to adjust the position of the projected image to be aligned with the user's pupil.

15. A method comprising:
providing an image from an image source;
conveying a beam of light through an optical arrangement to enable an image from the image source to be projected onto the user's eye wherein the optical arrangement comprises at least one light guide and an exit pupil where the exit pupil is arranged to be positioned proximate to an eye of a user and the at least one light guide is arranged to convey a beam of light from the image source to the exit pupil;
detecting the image projected onto the user's eye; and
enabling the detected image to be analysed so as to identify changes in the projected image.
